# EUROPEAN PATENT APPLICATION

(11) **EP 3 991 711 A1**
(43) Date of publication of application: **04.05.2022**
(21) Application number: 20204487.1
(22) Date of filing: 28.10.2020
(51) Int. Cl.: A61J 1/00, A61K 38/18, B65D 85/00

(54) **PHARMACEUTICAL PACKAGES COMPRISING POLYPROPYLENE CONTAINERS AND NGF AQUEOUS FORMULATIONS PACKAGED THEREIN**

(71) Applicant: Dompe' Farmaceutici S.P.A., 20122 Milan (IT)
(72) Inventor: ALLEGRETTI, Marcello, 67100 L'Aquila (IT); Apparente, Lucia, 67100 L'Aquila (IT); GENTILE, Marco Maria, 67100 L'Aquila (IT); MATTIOLI, Simone, 67100 L'Aquila (IT)
(74) Representative: Mauri, Elisabetta Maria Ester

(57) **Abstract**

The present invention relates to a pharmaceutical package comprising a plastic container and an aqueous formulations of nerve growth factor (NGF), in which at least the surface of the container in contact with the NGF aqueous formulation is made of particular polypropylenes (PPs), characterized by certain thermal properties and crystallinity. Advantageously said containers show a NGF adhesion significantly lower than that of siliconized glass or of other polypropylenes. It is thus possible to store aqueous formulations of NGF in less severe conditions, avoiding additional coating treatments to the containers and additives while keeping the NGF titer constant.

## Description

The present invention relates to pharmaceutical packages comprising particular polypropylene containers and a NGF aqueous formulations packaged therein. Packaging is one of the most important aspects of the pharmaceutical industry as it ensures protection and containment of the drug during transfer, storage, sale and use.

Primary packaging is of greatest significance as it is in direct contact with the drug product, so it needs to be inert and not to cause any alteration in its composition.

The type of primary packaging depends on the dosage form and the chemical composition of the drug. For liquid drug dosage forms, high quality glass or plastic materials are typically used. Glass is a widely used material because it is chemically inert and allows a very easy inspection of the content in the container. However, glass is fragile and expensive.

As regards plastics, they have the advantage of being flexible, low-weight, and cost-effective but in some cases issues arise due to the fact that some drugs could react with the container.

For pharmaceutical primary packaging, polypropylene based materials have been approved by Pharmacopeias and are commonly used.

Nerve growth factor (NGF) is a complex protein regulating the survival, development and function of neurons, both at a central and peripheral level.

NGF has been suggested to be effective for treating certain degenerative diseases of both the peripheral and central nervous systems, including hereditary sensory and motor neuropathies, hereditary and sporadically occurring system degeneration, amyotrophic lateral sclerosis Parkinson's disease, and Alzheimer's disease. Nowadays, NGF also finds a specific therapeutic use in the treatment of corneal diseases such as neurotrophic keratitis. NGF currently used in therapy is extracted from mouse submaxillary glands or manufactured by recombinant technologies.

The use of NGF in therapy poses some problems as its bioactivity, which depends on its secondary and tertiary structure, may be altered during manufacturing, purification or storage. NGF solutions may be subjected to protein aggregation that may result in titer variability.

In addition to problems of stability, like many other proteins, NGF binds nonspecifically to surfaces and to a variety of materials, including glass and plastics, with which NGF may come in contact during manufacture, storage or administration. This phenomenon may lead to a reduction in the concentration of the active in the solution and, consequently, to variable or insufficient amounts of NGF finally administered to the patient.

NGF is currently marketed either in freeze-dried form, to be reconstituted at the time of use, or as aqueous formulation that requires storage at low temperatures, usually -20°C, to minimize NGF aqueous formulations degradation and adhesion. Furthermore, in order to mitigate the adhesion of NGF to primary packaging, the inner contact surface of NGF currently marketed glass vials is covered with a coating that reduces the adsorption of proteins.

WO95/05845A1 describes NGF formulations and illustrates the drawbacks of scarce stability and undesired adhesion to various surfaces of this protein. To reduce or prevent NGF adhesion, this document suggests using stabilizing additives for the NGF aqueous formulations, such as surfactants like polysorbates, e.g. Tween^{®} 80, poloxamers as Pluronic^{®} F68, or proteins, such as albumin. Examples 5 and 6 describe stability studies of NGF aqueous formulations packaged in polypropylene drop-tip vials. The polypropylene of these vials is a generic uncharacterized material.

In the light of the above, there is still the need to maintain the activity and titer of NGF aqueous formulations during handling and storage without having to resort to the combined use of additives in the solution, to low temperatures all along manufacture and storage steps and to sophisticated and expensive containers for primary packaging.

### SUMMARY OF THE INVENTION

The Applicant has undertaken studies aimed at effectively preventing or minimizing the adhesion of NGF from aqueous formulations to the inner contact surface of the containers in which they are stored and has surprisingly found that particular polypropylenes (PPs), characterized by certain thermal properties and crystallinity, show a NGF adhesion significantly lower than that of siliconized glass or of other polypropylenes.

By using these particular PPs, it is thus possible to store aqueous formulations of NGF in less severe conditions, even at room temperature, advantageously avoiding additional coating treatments to the containers and stabilizing additives while keeping the NGF titer constant.

It this thus an object of the present invention a pharmaceutical package comprising a plastic container as primary packaging and a NGF aqueous formulation packaged therein, in which the plastic container has an inner surface in contact with the NGF aqueous formulation and an outer surface, wherein at least said inner contact surface is substantially made of at least a propylene-ethylene copolymer having a melting enthalpy ΔHm less than 95 J/g, measured by DSC according to the method reported in the description.

A further object of the present invention is a method for selecting a propylene-ethylene copolymer suitable for manufacturing at least the inner contact surface of the plastic container of the pharmaceutical package of the invention that comprises:
- providing at least a propylene-ethylene copolymer sample
- measuring the melting enthalpy ΔHm of the propylene-ethylene copolymer sample by DSC according to the method reported in the description,
- selecting the propylene-ethylene copolymer as suitable if its melting enthalpy ΔHm is less than 95 J/g.

A further object of the invention is the use of the above defined plastic container for packaging a NGF aqueous formulation.

### DEFINITIONS

With the term "polypropylene", both homopolymers and copolymers of propylene are meant.

With the acronym "PP", polypropylene is meant.

With the term "propylene based material", a material comprising a propylene mer % higher than 50% is meant.

With the term "propylene - ethylene copolymer" a copolymer of propylene with ethylene is meant, in which the propylene monomer is the majority monomer. The term "propylene-ethylene copolymer" herein also includes propylene - ethylene terpolymers in which the propylene monomer is the majority monomer and an additional olefin monomer is present.

With the term "majority monomer", a monomer present in the polymer in amount higher than 50% mol is meant.

With the term "mer", a repeating unit part of a polymer is meant.

With the acronym "NGF", nerve growth factor is meant.

With the term "substantially made of" a percentage of the component in re higher than 80% by weight is meant.

With the term "liquid drug dosage form" the liquid formulation in which the drug product is manufactured and made available for use - such as a solution, an emulsion, a suspension, a lotion and the like is meant.

With the term "primary packaging" or "primary container", the packaging in direct contact with the liquid drug dosage form is meant.

With the term "mono-dose package", a package including a single dose of the drug is meant.

With the term "multi-dose package", a package including more than a single dose of the drug is meant.

With the term "contact surface" the surface of the plastic container used in contact with the NGF aqueous formulation is meant.

Unless otherwise stated, herein all the percentages are percentages by weight.

### BRIEF DESCRIPTION OF THE FIGURES

- Figure 1 shows the bar charts of contact angles of formulations F1 to F5 on the tested PP materials PP1 to PP5;
- Figure 2 shows the graph of frequency of oscillation of the electrode vs time, measured on a Quartz Crystal Microbalance (ΔF - Hz vs time -min., 6 replicas), in an experiment of adsorption of rhNGF from formulation F1 onto a film of PP2 material;
- Figures 3 and 4 show the graphs of photoelectron emission of NGF adsorbed on the surface of PP3, PP4 and PP5 specimens from formulation F6;
- Figure 5 shows the bar charts of absorbance (at 280 nm) of formulation F7 vs control formulation (Ctrl), the formulation F7 packaged in double siliconized glass vials or in PP3 bottles and stored at temperatures of 2-8°C, 25°C and 37°C; absorbance measured at time points of 1 week, 2 weeks, 1 month, 2 months and 3 months (3 months stability test).
- Figure 6 shows the bar charts of tryptophan fluorescence (Intensity at λ max - x 10⁷ cps) of formulation F7 vs control formulation (Ctrl), the formulation F7 packaged in double siliconized glass vials or in PP3 bottles and stored at temperatures of 2-8°C, 25°C and 37°C; fluorescence measured at time points of 1 week, 2 weeks, 1 month, 2 months and 3 months (3 months stability test).

Unless otherwise indicated, all the percentages are expressed as percentages by weight.

### DETAILED DESCRIPTION OF THE INVENTION

An object of the present invention is pharmaceutical package comprising a plastic container and a NGF aqueous formulation packaged therein, in which the plastic container has an inner surface in contact with the NGF aqueous formulation and an outer surface, wherein at least said inner contact surface is substantially made of at least a propylene-ethylene copolymer having a melting enthalpy ΔHm less than 95 J/g, measured by DSC according to the method reported in the description.

The plastic container of the package of the invention is a plastic container suitable for primary packaging of NGF aqueous formulation.

The plastic container of the package of invention can be a rigid or a flexible container.

The container of the package of invention can be an ampoule, a vial, a bottle, a jar, a bag or a pouch.

Preferably, the present container is a bottle or a vial.

The plastic container of the package of invention can have a monolayer or a multilayer structure, preferably has a monolayer structure.

In case of a monolayer structure, the plastic container is substantially made of a propylene-ethylene copolymer as herein defined, preferably it comprises at least 90%, more preferably at least 95%, even more preferably at least 99% of at least a propylene-ethylene copolymer as herein defined.

In case of a multilayer structure, at least the contact surface layer is substantially made of at least a propylene-ethylene copolymer as herein defined, preferably it comprises at least 90%, more preferably at least 95%, even more preferably at least 99% of at least a propylene-ethylene copolymer as herein defined.

In one embodiment, the whole container is substantially made of at least a propylene-ethylene copolymer as herein defined, preferably it comprises at least 90%, more preferably at least 95%, even more preferably at least 99% of at least a propylene-ethylene copolymer or mixtures of them, as herein defined.

The complement to 100% of the contact surface layer or of the whole container can be represented by other preferably miscible thermoplastic polymers such as for instance ethylene - vinyl acetate copolymer and the like.

The present plastic container is characterized by at least a propylene - ethylene copolymer forming at least the contact surface layer, said propylene - ethylene copolymer having a melting enthalpy ΔHm less than 95 J/g, measured by DSC according to the method reported in the description.

Preferably, the propylene - ethylene copolymer has a melting enthalpy ΔHm less than 90 J/g, more preferably less than 80 J/g.

Preferably, the propylene - ethylene copolymer has a melting enthalpy ΔHm greater than 35 J/g, more preferably greater than 40 J/g.

Preferably, the melting enthalpy ΔHm of the propylene - ethylene copolymer is between 90 J/g and 35 J/g, more preferably between 80 J/g and 40 J/g.

A 100% crystalline propylene homopolymer, which is not suitable for manufacturing the container of the present package, has a melting enthalpy ΔHm of about 207 J/g (Thermal Application Note - Polymer Heats of Fusion - Roger L. Blaine , TA Inctruments, 109 Lukens Drive, New Castle DE 19720, USA).

Preferably, the propylene - ethylene copolymer has a melting temperature Tm lower than 160°C, more preferably lower than 155°C, measured according to the DSC method reported in the description.

Preferably, the propylene - ethylene copolymer has a melting temperature Tm higher than 130°C, more preferably higher than 140°C, measured according to the DSC method reported in the description.

Preferably, the propylene - ethylene copolymer has a melting temperature Tm between 160°C and 130°C, more preferably between 155°C and 140°C, measured according to the DSC method reported in the description.

Preferably, the propylene - ethylene copolymer has a Melt Flow Index less than 10 gr/10 min, more preferably less than 9 gr/10 min, measured according to ISO 1133 at 230°C and 2.16 kg.

Preferably, the propylene - ethylene copolymer has a Melt Flow Index between 1 and 20 gr/10 min, measured according to ISO 1133 at 230°C and 2.16 kg. Preferably, the propylene - ethylene copolymer is a random propylene - ethylene copolymer.

Preferably, the propylene - ethylene copolymer is semi-crystalline.

Preferably, the content of ethylene monomer in the propylene - ethylene copolymer is less than 40%, more preferably less than 30%, and at least 5%.

In one embodiment, the propylene - ethylene copolymer is a terpolymer of propylene, ethylene and an alpha-olefin, preferably butene.

Preferably, the propylene - ethylene copolymer contact surface - that in the final container is in contact with the NGF aqueous formulation - is characterized by a surface roughness Sa, before NGF incubation, lower than 25 nm, more preferably lower than 20 nm, even more preferably lower than 15 nm, measured according to ISO 4287 (1997).

Preferably, the propylene - ethylene copolymer surface that in the final container is in contact with the NGF aqueous formulation is characterized by a contact angle greater than 90°, preferably greater than 95°, when measured in respect of the solution F1 as described in the present experimental part.

Preferably, the propylene-ethylene copolymer is approved in at least a Pharmacopeia.

Examples of propylene-ethylene copolymers suitable for the manufacture of the container of the package of the invention are the copolymers of the Bormed^{™} series of Borealis, in particular the propylene-ethylene copolymers Bormed^{™} RD808CF, Bormed^{™} SB815MO and Bormed^{™} SC876CF.

The present plastic container may comprise a single propylene - ethylene copolymer or an admixture of two or more propylene - ethylene copolymers.

In case of an admixture of propylene - ethylene copolymers, they are miscible to provide a monophasic homogeneous blend with a single melting peak and a single melting enthalpy ΔHm, according to the preferences expressed above.

In the present plastic container, in addition to the plastic polymers, minor amounts of additives commonly used in this field may be present.

Depending on the container, different known manufacturing techniques can be used, in which the present propylene - ethylene copolymers is the material for the entire wall of the container or at least for the contact surface layer.

For instance, rigid thicker containers (e.g. bottles or vials) can be made by injection moulding the selected polymers while flexible thin containers (e.g. bags and pouches) by suitably cutting and self-sealing mono or multilayer films, as known in the art of packaging.

Advantageously, the pharmaceutical package of the invention provides for storage of NGF aqueous formulations under easier storage conditions meanwhile minimizing NGF adhesion on the contact surface of the container and preserving NGF titer.

In particular, the pharmaceutical package of the invention allows to preserve the aqueous formulation of NGF at temperatures higher than 4°C, even at room temperature, without using stabilizing additives of the formulation, for example that prevent adhesion or aggregation of NGF.

Preferably, the container is a vial or a bottle that, at least at the inner contact surface comprises, preferably consists of, a propylene - ethylene copolymer as previously defined.

The present pharmaceutical package comprises a NGF aqueous formulation, such as an aqueous solution, emulsion, suspension, lotion and the like, preferably a NGF aqueous solution, more preferably a NGF aqueous solution for ophthalmic use. The NGF aqueous formulation comprises NGF in therapeutically effective amounts. Preferably, in the aqueous formulation, NGF is present in amount from about 0.0001 % to about 0.5% w/v, more preferably from about 0.001 % to about 0.1 % w/v, most preferably of about 0.002% w/v of the aqueous formulation.

The NGF aqueous formulation may contain a sufficient amount of biologically acceptable salts to provide the correct fluid tonicity and to maintain the NGF in solution.

Preferably, the NGF aqueous formulation contains sufficient salt to be isotonic, within physiologically acceptable limits, with eye fluids, human blood or cerebral spinal fluid, depending on the therapeutic use.

The preferred salt is sodium chloride (NaCl) but other biologically acceptable salts may be used, such as for instance potassium chloride (KCI), calcium chloride (CaCl₂) and magnesium chloride (MgCl₂) and their admixtures.

The NGF aqueous formulation further contains a biologically acceptable buffer to provide and maintain the correct pH during storage.

The NGF aqueous formulation is buffered with a biologically acceptable buffer to a pH between about 6.8 to about 7.5, more preferably to a pH of about 7.2.

The preferred buffer is phosphate buffer, but other buffers capable of maintaining the pH within the desired range, especially buffers suitable for ophthalmic use, are also included. The preferred amount of buffer will vary depending on the type of buffer used and its buffering capacity.

The NGF aqueous formulation may optionally include additives that further reduce or prevent NGF adsorption to various surfaces, preferably surfactants such as for instance polysorbates such as Tweeny 80, poloxamers such as Pluronics F68, or proteins such as serum albumin.

In one embodiment, the NGF aqueous formulation does not include any additives reducing adsorption, preferably does not include any surfactant.

Excipients commonly used in pharmaceutical aqueous formulations and known to the technical expert, such as sugars, sugar alcohols, aminoacids, cellulose-derivatives, polyethylene glycols, may be present in the NGF aqueous formulation. The NGF aqueous formulation comprises water in an amount sufficient to achieve the appropriate concentration of formulation components.

NGF herein includes any form of nerve growth factor, from any source, providing that it exhibits biological activity and binds to the NGF receptors.

NGF also comprises modified forms of NGF that retain NGF bioactivity and receptor binding, including fusion proteins, NGF fragments and mutants in which certain amino acids have been deleted or replaced while maintaining sufficient NGF bioactivity and receptor binding to provide therapeutic activity.

In one embodiment, NGF is of murine source.

Preferably, NGF is human NGF (hNGF), more preferably it is recombinant hNGF (rhNGF).

Methods of obtaining rhNGF, suitable for use in the present aqueous formulation, are known to those skilled in the art, for instance the processes described in WO9505845A1, WO0022119A1 and WO2013092776A1.

The NGF of the present aqueous formulation preferably has a purity higher than 70%, more preferably higher than 80%, even more preferably higher than 90% and most preferably higher than 95%. The purity of isolated NGF for use in the present aqueous formulations may be determined by conventional means known to those skilled in the art.

The present NGF aqueous formulation is preferably prepared in advance and then packaged and stored in the container of the invention.

However, in one embodiment, the NGF formulation is packaged in the container in a powdery form e.g. in a lyophilized form, then upon reconstitution with an aqueous reconstituting vehicle, stored as a NGF aqueous formulation up to administration to patients in need of therapy.

The container of the package of invention after conventional filling with the NGF formulation needs to be closed.

Closures, used for closing drug containers after the filling process, should be as inert as possible. They should not give rise to undesired interactions between the contents and the outside environment, and should provide a complete seal. Besides their protective function, closures must also allow the easy and safe administration of the drug.

Examples of closures suitable for the present container are simple elastomeric caps or they are part of more sophisticated delivery systems such as for instance the Novelia^{®} system by Nemera or the Ophthalmic Squeeze Dispenser (OSD) by APTAR.

Advantageously the present package, if provided by sufficient gas-barrier properties, may comprise a modified atmosphere, such as an oxygen-free atmosphere, with the aim to improve the stability under storage.

In such a case, the package should be gas-tight, possibly including suitable gas barrier materials as known in the art of packaging.

Preferably, the NGF aqueous formulation is an NGF aqueous solution for ophthalmic use, in form of eye drops administered by ocular route.

A preferred NGF aqueous formulation in addition to NGF, preferably rhNGF, comprises trehalose, mannitol, Methocel, PEG 6000, L-methionine, phosphate buffer, water and has a pH of about 7.2.

The present pharmaceutical package can be a mono-dose package or a multi-dose package.

In one embodiment, the pharmaceutical package of the invention is a mono-dose package, namely a pre-loaded sterile plastic applicator comprising a container as previously defined and an NGF aqueous solution, preferably not including stabilizing addittives, packaged therein, for a single administration.

In one embodiment, the pharmaceutical package of the invention is a multi-dose package, comprising a multi-dose sterile container as previously defined and a NGF aqueous solution, preferably not including stabilizers, for multiple administration, preferably by using delivery systems.

The NGF aqueous formulations are useful for treating individuals with conditions responsive to NGF therapy.

Typically, such formulations are sterile, and are suitable for ophthalmic, intravenous, intramuscular, parenteral or intracerebroventricular administration, preferably for ophthalmic administration.

Such therapy may be useful to treat corneal diseases such as neurotrophic keratitis or neuronal dysfunctions involving neuronal injury or degeneration of NGF responsive neurons such as in Alzheimer's disease.

The NGF aqueous formulation as treatment for neurotrophic keratitis is typically administered by ocular route.

The NGF aqueous formulation as treatment for dementia may be administered by any of a variety of routes depending on the specific end use.

To overcome the difficulties presented by the blood-brain barrier, NGF may be administered into the CNS by direct intraventricular injections or via drug impregnated implants or pumps. Another administration route is by continuous infusion through an intracerebroventricular cannula device.

Advantageously, at least the surface of the plastic containers that gets in contact with the NGF aqueous formulation during administration, is substantially made of a propylene-ethylene copolymer as previously defined.

The exact dose and regimen for administration will depend upon many factors, such as the route of administration and the degree of affliction of the individual receiving treatment.

The present invention is better illustrated in the following experimental section.

### EXPERIMENTAL PART

In this experimental section, the interaction between the protein (rhNGF) and the plastic material (polypropylene PP) was evaluated both in qualitative and quantitative terms.

Different analytical techniques were used to determine directly and indirectly the adsorption of rhNGF to the PP packaging material.

It has been surprisingly found that different degrees of crystallinity of the PP material resultin different levels of NGF adsorption and that the tested analytical techniques provided congruent and robust, interrelated results.

In these tests, we used the pharmaceutical grade PP materials listed in Table 1, already approved for the manufacture of drugs primary containers:

**Table 1: PPs**

| n. | Manufacturer | Commercial Code | Notes |
|---|---|---|---|
| PP1 | Borealis | Bormed^{™} RD808CF | Polypropylene Random Copolymer, Density 0.905 g/cc (ISO1183), Melt Flow Index 8.0 g/10 min (2.16 kg, 230°C, ISO1133), Melting temperature 140°C (DSC, ISO 3146), Vicat Softening Point 125°C (1.02 kg, ISO306) |
| PP2 | Borealis | Bormed^{™} HD810MO | Polypropylene Homopolymer, Melt flow index 10 g/10 min ISO 1133 (230°C, 2.16 kg), Melting temperature (10°C/min) 164°C ISO 11357-1/-3 |
| PP3 | Borealis | Bormed^{™} SB815MO | Polypropylene Random Copolymer, Melt Flow Index 1.5 g/10 min (230°C/2.16 kg) ISO 1133, Melting temperature (DSC) 145°C ISO 11357-3 |
| PP4 | Borealis | Bormed^{™} SC876CF | Polypropylene Random Copolymer, Melt Flow Index (230 °C/2.16 kg) 3.8 g/10 min ISO 1133, Melting temperature (DSC) 149 °C ISO 11357-3 |
| PP5 | Lyondell Basell | Purell HP372P | Polypropylene Homopolymer, Melt Flow Index (230°C/2.16 kg) 18 g/10 min, Density (23°C) 0.90 g/cm³, Vicat Softening Temperature (A50) 150°C |

Injection moulding tests showed that the PP materials were all easily machinable and met the specifications required for extrusion and moulding process in terms of formability and mechanics.

Specimens to carry out the tests were manufactured for each material by injection moulding. The specimens were lozenges 15 cm long, 2 cm wide, 1 cm high.

### Example 1

### Measurement of melting enthalpy (ΔHm) and melting temperature (Tm) of PPs by Differential Scanning Calorimetric (DSC) Analysis

With the aim to evaluate the thermal properties of different PPs, Differential Scanning Calorimetric Analysis was carried out with a DSC TA 2010 instrument, operating by double thermal scan (first heating from 0°C to 200°C at a speed of 20°C/min, intermediate cooling from 200°C to 0°C at a speed of 20°C/min and second heating from 0°C to 200°C at a speed of 20°C/min). Two specimens for each material were tested.

The average results of the DSC analysis are reported in the following Table 2:

**Table 2: PPs thermal properties**

| PP Sample | First heating | | | Second heating | | |
|---|---|---|---|---|---|---|
| | Tm onset (°C) | Tm peak (°C) | ΔHm(J/g) | Tm onset (°C) | Tm peak (°C) | ΔHm (J/g) |
| PP1 | 120 | 144 | 61 | 126 | 144 | 75 |
| PP2 | 149 | 165 | 86 | 148 | 164 | 100 |
| PP3 | 121 | 151 | 52 | 137 | 151 | 58 |
| PP4 | 127 | 152 | 40 | 139 | 151 | 41 |
| PP5 | 148 | 163 | 84 | 152 | 163 | 97 |

The relevant melting enthalpy ΔHm (J/g) and Melting Temperature (Tm peak °C) were those measured at the second heating.

As the degree of crystallinity of plastic materials is proportional to the respective melting enthalpy ΔHm (J/g), the tested PPs could be ordered by crystallinity as shown below (from left to right, from the most to the least crystalline):
PP2 > PP5 > PP1 >PP3 >PP4

### Example 2

### Measurement of Contact Angle

Five rhNGF water-based formulations (F1 - F5) were used for this test. The formulations of F1 - F5 are shown in Table 3 below:

**Table 3: formulations**

| | rhNGF conc. (µg/ml) | Other ingredients | Notes |
|---|---|---|---|
| F1 | 20 | phosphate buffer (50 mM) methionine (0.010 mg/ml), NaCl 100 mM, water q.s. | pH 7.2 |
| F2 | 20 | Trehalose dihydrate 5.4 mg/ml, Mannitol 13.1 mg/ml, Methocel K4M 1.07 mg/ml, PEG 6000 10.7 mg/ml, L-methionine 0.010 mg/ml, phosphate buffer 50 mM, water q.s | pH 7.2 |
| F3 | 20 | phosphate buffer (50 mM), surfactant 0.107% w/v methionine 0.020 mg/ml, NaCl 100 mM, water q.s. | pH 7.2 |
| F4 | 20 | phosphate buffer (50 mM), surfactant 0.053% w/v methionine 0.020 mg/ml, NaCl 100 mM, water q.s. | pH 7.2 |
| F5 | 20 | phosphate buffer (50 mM), surfactant 0.0214% w/v, methionine 0.020 mg/ml, NaCl 100 mM, water q.s. | pH 7.2 |

| | | | |
|---|---|---|---|
| q.s. quantum sufficit; surfactant was a poloxamer suitable for ophthalmic use. | | | |

Formulations F1 - F5 were prepared as described herein below:
70 ml of water for injection (W.F.I.) were introduced in a 150 ml beaker, then all the ingredients but the rhNGF were added and kept under magnetic stirring up to complete dissolution. The exact amount of rhNGF was added and the solution was brought up to volume (100 ml) with W.F.I. under stirring. The pH of the solution was measured and, in case, corrected with sodium hydroxide or hydrochloric acid.

Formulation F1 was the one expected to show the highest rhNGF deposition as it contained no surfactants or other stabilizing additives.

The contact angle measurements were made with a Dataphysics - Contact Angle System OCA 200 instrument, with an electronic control software with automatic alignment. Ten contact angle measurements have been made for each material and then the mean value and the standard deviation calculated. In order to minimize the error induced by the different roughness of the sample surfaces generated by the injection moulding process, five measurements were taken on the front face and five measurements on the back face of each sample.

For each formulation F1 - F5, a total of 10 drops of 3 µl each were deposited on each PP sample. The deposited drops were not stable over time, as they tended to spread on the surface of the support. Hence, the image of the drop for the acquisition of contact angles was taken within 10 seconds of the deposition of the drop. Average contact angles and standard deviation values were recorded for each formulation F1-F5 for materials PP1 - PP5 and reported in Table 4 below:

**Table 4: contact angles (°)**

| Formulation | PP1 | PP2 | PP3 | PP4 | PP5 |
|---|---|---|---|---|---|
| F1 | 98 ± 2 | 74 ± 2 | 102 ± 1 | 97 ± 2 | 83 ± 0,5 |
| F2 | 77 ± 2 | 70 ± 3 | 88 ± 1 | 84 ± 2 | 73 ± 3 |
| F3 | 82 ± 2 | 75 ± 2 | 86 ± 1 | 84 ± 2 | 80 ± 5 |
| F4 | 79 ± 1 | 78 ±2 | 85 ± 3 | 82 ± 1 | 81 ± 1 |
| F5 | 80± 3 | 82± 1 | 93± 1 | 86± 3 | 81 ± 1 |

According to the values of contact angles, the tested PP materials were characterized by the following order of wettability (from left to right, from the most wettable to the least wettable):
PP2 > PP5 > PP1 >PP4>PP3

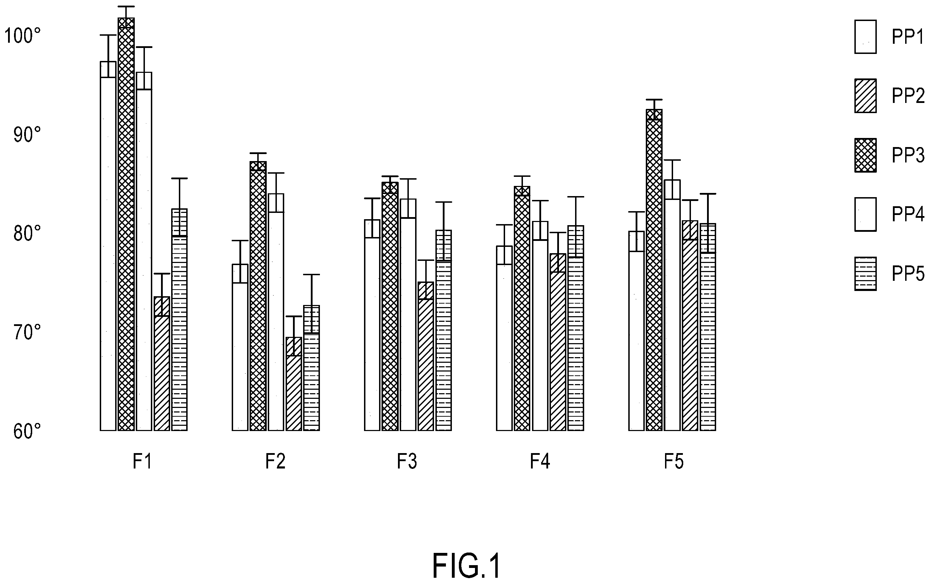

The trend of the average contact angle, as a function of the formulation F1 - F5 and the specimen material, was reported in Figure 1.

With reference to Table 4 and Figure 1, we observed a similar behavior for some of PP materials. In particular PP3 and PP4 were the ones with the lowest wettability (highest contact angle), especially with formulation F1 (rhNGF in buffer formulation pH7.2 - the most discriminating formulation as devoid of surfactants and other additives). From DSC analysis, PP3 and PP4 were the ones with lowest crystallinity (i.e. the lowest ΔHm, see Table 2).

On the other hand, the materials PP5 and PP2 had higher wettability - namely a more hydrophilic surface - and a higher crystallinity than the former.

In conclusion, this test showed that there was a correlation between, on the one hand, the crystallinity and the ΔHm of PPs and, on the other hand, their wettability and contact angle. A greater contact angle was indicative of a lower wettability of the plastic surface, which, in turn, was predictive of less NGF adsorption, as confirmed by the following adsorption tests.

### Example 3

### Measurement of rhNGF adsorption on PP samples

The technique adopted for this test used a Quartz Crystal Microbalance (QCM) and provided a direct and quantitative analysis of the degree of adsorption of rhNGF on the material.

The PP plastic material to be tested (PP1 to PP5) was first dissolved in decalin (2% w/v) under stirring at a temperature of 150°C for 1 hour and then deposited by electrospinning as a thin film on a quartz crystal electrode (spin coating at 3500 rpm). The equipment for transferring the dissolved polymer to the spin coater were pre heated at 120°C - 150°C. The thickness of the PP films is reported in the following Table 5:

**Table 5: thickness of PP films**

| Sample | Thickness (nm) |
|---|---|
| PP1 | 163 ± 8 |
| PP2 | 400 ± 21 |
| PP3 | 280 ± 15 |
| PP4 | 200 ± 12 |
| PP5 | 400 ± 20 |

The coated electrodes were incubated overnight in PBS prior to protein adsorption experiments. The following formulation F6, prepared as formulations F1-F5, was used:

**Table 6: rhNGF formulation**

| Formulation | rhNGF conc. (µg/ml) | Other ingredients | Notes |
|---|---|---|---|
| F6 | 20 | phosphate buffer (50 mM) methionine (0.020 mg/ml), NaCl 100 mM, water q.s. | pH 7.2 |

The formulation was pumped with a high precision multi-channel peristaltic pump through a Teflon tube of 0.75 mm of inner diameter and applied onto the coated electrode, at a flow of 50 microliters / sec.

A voltage difference ΔV was applied to the two sides of the electrode, which began to vibrate at a certain frequency.

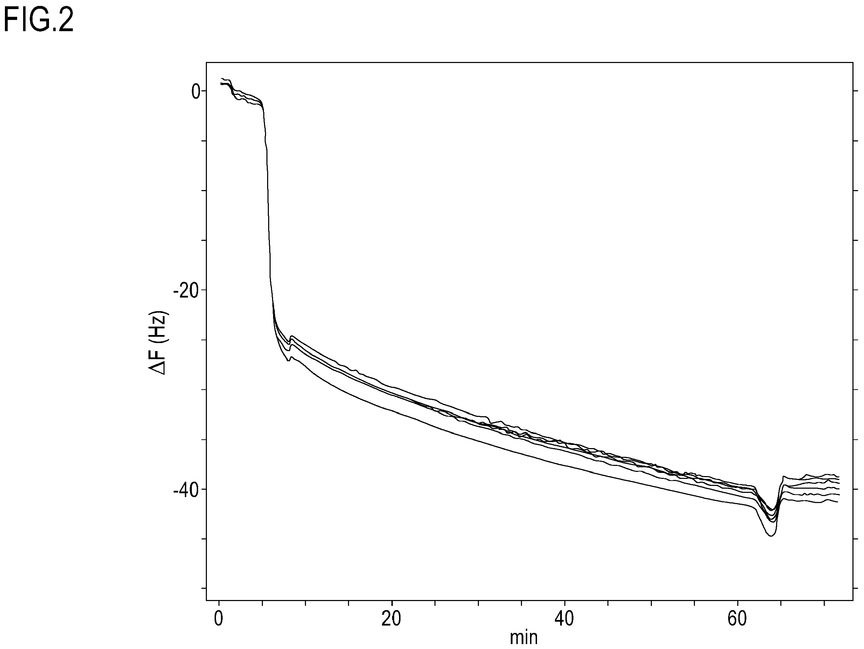

The frequency of oscillation of the electrode decreased, as the rhNGF film deposited on it increased, up to an adsorption plateau after about 60 minutes, as shown in the graph of Figure 2 relating to PP2 material.

Tables 7 and 8 below show the adsorption of rhNGF, before and after washing the electrode with phosphate buffered saline (PBS), measured as mass (ng/cm²) once it reached the adsorption plateau after about 60 min.

**Table 7**

| Adsorbed rhNGF before washing with PBS | | |
|---|---|---|
| PP sample | ΔF (Hz) | Mass (ng/cm²) |
| PP1 | -28.5 ± 1.0 | 503.7 ± 17.7 |
| PP2 | -38.9 ± 0.7 | 687.9 ± 17.2 |
| PP3 | -30.9 ± 0.9 | 546.1 ± 16.0 |
| PP4 | -29.0 ± 1.2 | 529.6 ± 20.9 |
| PP5 | -121.9 ± 2.5 | 2158.2 ± 43.7 |

The electrode was washed with PBS to verify the stability of rhNGF adsorption on the surface of the different PPs.

**Table 8**

| Adsorbed rhNGF after washing with PBS | | |
|---|---|---|
| PP sample | ΔF (Hz) | Mass (ng/cm²) |
| PP1 | -24.3 ± 0.7 | 430.9 ± 13.1 |
| PP2 | -38.5 ± 1.3 | 627.1 ± 23.1 |
| PP3 | -30.9 ± 0.7 | 546.6 ± 12.1 |
| PP4 | -29.0 ± 0.5 | 514.94 ± 9.4 |
| PP5 | -120.7 ± 2.3 | 2136.2 ± 41.3 |

From the analyses above, we observed that the most crystalline and hydrophilic PP materials (PP5 and PP2) had a higher mass of rhNGF adsorbed on the surface, compared to the most hydrophobic PP materials with lower crystallinity (PP3, PP4 and PP1).

Post-washing results indicate that the adsorbed rhNGF is quite stable on the plastic surface and that, on all the surfaces, the percentage of desorbed rhNGF after washing was rather low.

In conclusion, the results of the adsorption test correlated well with the contact angle and crystallinity data, showing a lower NGF adsorption on less crystalline, more hydrophobic materials.

### Example 4

### Measurement of surface roughness by atomic force microscopy (AFM)

Atomic force microscopy is a technique that allows to measure, through the deflection of a small lever called "cantilever", minimum forces present on a surface and provide a three-dimensional view of the same surface with a resolution between 1 and 10nm.

The AFM detector measures the deflection of the cantilever and converts it into an electrical signal. The intensity of this signal is proportional to the shift of the cantilever.

### Part 1

A drop of the formulation F6 containing the protein (rhNGF) was deposited onto the surface of the lozenge specimens of PP1 - PP5 (one specimen for each PP) and incubated for 1 week at 4°C.

After incubation, the specimens were rinsed and immersed in a first beaker containing 200 ml of Milli-Q water for 5 min and then transferred in a second beaker containing 200 ml of Milli-Q water too, for another 5 min. The surface was then dehydrated under a weak stream of nitrogen. This procedure was necessary to remove the NGF, not adsorbed but present in the solution entrapped on the surface of the PP, and salts that would induce artifacts once dried on the sample.

The surface roughness was measured, before and after incubation with the rhNGF formulation, according to ISO 4287 (1997). The samples were analyzed by acquiring the surface scan images with an Atomic Force Microscope (Bruker), working in dynamic mode. TESPA levers (Bruker, USA) with elastic constant of 40 N/m and resonance frequency of 300 kHz were used. The radius of curvature of the tip was about 10 nm.

Images were acquired at different scales, from 50 to 2 µm.

The results, expressed as Sa, arithmetic mean of the absolute of the ordinate values within a definition area (A), are shown in the following Table 9:

**Table 9**

| Roughness by AFM | | | | | |
|---|---|---|---|---|---|
| | PP1 | PP2 | PP3 | PP4 | PP5 |
| Sa (nm) before NGF incubation | 12 ± 3 | 10 ± 2 | 13 ± 7 | 14 ± 5 | 9 ± 2 |
| Sa (nm) after 1 week of NGF incubation | 16 ± 4 | 24 ± 5 | 10 ± 4 | 14 ± 3 | 29 ± 7 |
| Delta Sa (nm) | 4 ± 7 | 14 ± 7 | - 3 ± 11 | 0 ± 8 | 20 ± 9 |

Sa is the extension of Ra (arithmetical mean height of a line) to a surface. It expresses, as an absolute value, the difference in height of each point compared to the arithmetical mean of the surface.

From the Sa values of Table 9, we observed that for only two of the materials - PP5 and PP2 - the roughness increased considerably after incubation with the NGF (see Delta Sa -difference before and after 1 week of NGF incubation).

These roughness data correlated well with the adsorption data measured by Quartz Crystal Microbalance of Tables 7 and 8 and with the contact angles of Table 4, being PP2 and PP5 the most hydrophilic materials, having the roughest surface and causing the highest NGF adsorption.

The other materials PP1, PP3 and PP4 seemed poorly adsorbing the NGF and an increase in surface roughness after incubation could not be appreciated. Actually, PP3 material even seemed to reduce its surface roughness after incubation with NGF.

### Part 2

In the second part of the experiment, we discarded the less interesting hydrophilic materials (PP5 and PP2) and re-examined only the most promising hydrophobic materials (PP3, PP1 and PP4).

We also made a variation in the sample preparation methodology. The rhNGF formulation F6, this time was incubated for 48 h instead of 1 week. The roughness data are shown in Table 10 below:

**Table 10**

| Roughness by AFM | | | |
|---|---|---|---|
| | PP1 | PP3 | PP4 |
| Sa (nm) before rhNGF incubation | 12 ± 3 | 13 ± 7 | 14 ± 5 |
| Sa (nm) after 48 h of NGF incubation | 20 ± 4 | 14 ± 6 | 10 ± 3 |
| Delta Sa (nm) | 8 ± 7 | 1 ± 13 | - 4 ± 8 |

The data confirmed that among the most promising, hydrophobic, low-crystalline PP1, PP3 and PP4 polypropylenes, PP3 and PP4 showed a constant or even lower roughness after incubation with NGF.

### Example 5

### Measurement of Nitrogen on the surface of PP samples by X-ray photoelectron spectroscopy (XPS)

X-ray photoelectron spectroscopy is a spectroscopy technique used to probe the surfaces of materials. It allows to assess the chemical elements that make up the surface of a material and sometimes to determine their bonding state.

The electrons emitted by the material that has been hit by a monochromatic X-ray beam are expelled with a kinetic energy proportional to the bonding energy, indicative of the chemical element.

In this case, we analyzed the best hydrophobic materials PP3 and PP4, previously studied in terms of Contact Angle, AFM and QCM, comparing them with PP5, the worst material in terms of NGF adsorption.

### Part 1

A drop (about 45 microliter) of formulation F6, was deposited on the surface of the PP material and left for 5 seconds, then the surface was washed with water, dried under nitrogen flow and analyzed as described below.

All XPS spectra were measured on a Surface Science Instruments S-Probe spectrometer. This instrument had a monochromatic A1 Ka X-ray and a low-energy electron flood gun for charge neutralization of non-conducting samples. The samples were mounted onto double-sided tape and run as insulators. X-ray analysis area for these acquisitions was approximately 800 µm in diameter. Pressure in the analytical chamber during spectral acquisition was less than 5 x 10⁻⁹ torr. Pass energy was set at 150 eV. The take-off angle was 0° (0° take-off angle = 100 A sampling depth).

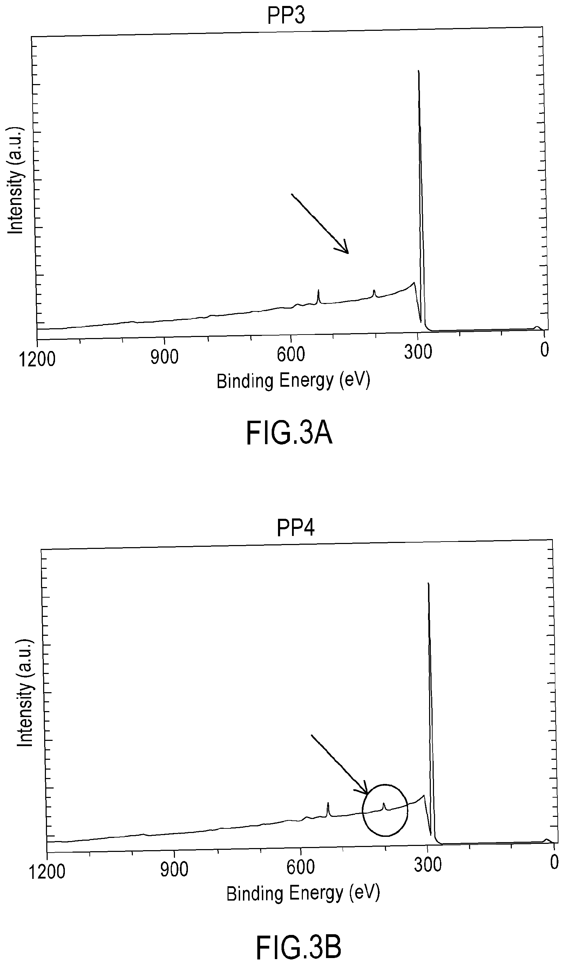

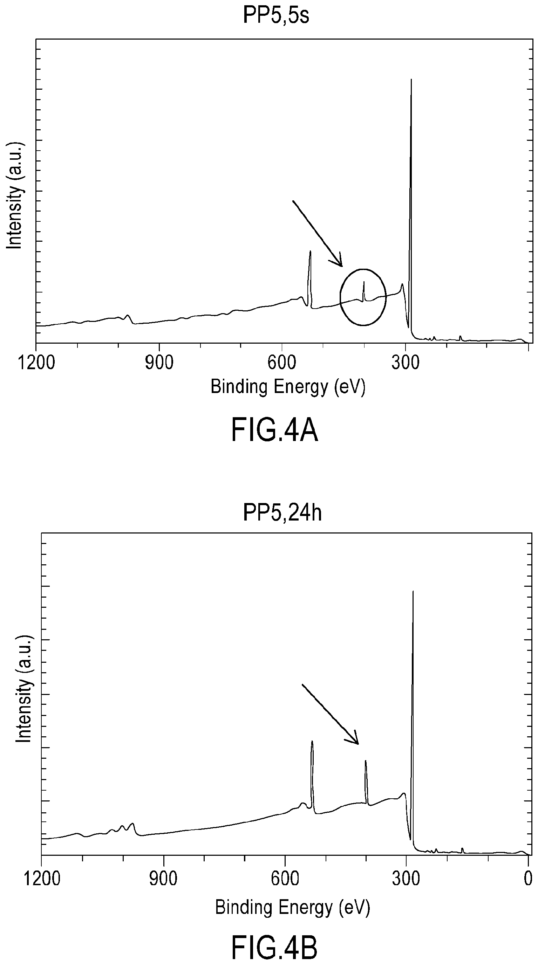

The graphs in Figures 3 and 4 show the photoelectron emission and, in particular, they highlight the peak of nitrogen emission, indicative of the presence of rh NGF on the surface.

It should be noted that for the more hydrophobic plastic materials with low crystallinity (PP3 e PP4, Figures 3A and 3B) the peak emission of the nitrogen atom had a significantly lower intensity (intensity 0.4 - 0.5 a.u.) compared to the peak associated with the more hydrophilic material with high crystallinity (PP5) (intensity 2 a.u.) of Figure 4A.

These results, that demonstrate a lower NGF deposition for the samples PP3 and PP4, correlated very well with those of the other previous techniques reported above.

### Part 2

We wanted to highlight the high adsorption property of PP5 material by incubating the NGF for a longer period of time (24 hours) and by evaluating the electron emission intensity associated with the nitrogen peak.

As can be seen from the graphs in Figure 4, the rhNGF continued to adsorb on the plastic surface over a 24-hour period to provide a peak with an emission intensity of about 4 a.u. (Figure 4B), which is twice as high as the initial 2 a.u. measured after 5 seconds of incubation (Figure 4A).

At the end of this first experimental phase, we concluded that:
1) Higher crystallinity polypropylenes, such as propylene homopolymers (PP2 and PP5), were associated with increased NGF surface adsorption.
2) Lower crystallinity polypropylenes, such as propylene - ethylene copolymers PP1, PP3 and PP4, resulted in lower surface adsorption of NGF.
3) Adsorption was rather stable and irreversible: once the NGF adsorbed to the plastic surface, desorption was very difficult to occur.

A summary of the main results collected in the above tests is reported in Table 11 below:

**Table 11: summary table**

| PP | ΔHm (J/g) ^{§} | Tm °C^{§} | Contact Angle° vs F1 | QCM Adsorbed mass (ng/cm²) | Delta AFM Roughnes s* Sa (nm) | XPS intensity N emission* | Overall score |
|---|---|---|---|---|---|---|---|
| PP 1 | 75 | 14 4 | 98 ± 2 | 503.7 ± 18 | 4 ± 7 | 0,4-0,5 | +++ |
| PP 2 | 100 | 16 4 | 74 ± 2 | 687.9 ± 17 | 14 ± 7 | 1,4-1,5 | - |
| PP 3 | 58 | 15 1 | 102 ± 1 | 546.1 ± 16 | -3 ± 11 | 0,2-0,4 | +++ |
| PP 4 | 41 | 15 1 | 97 ± 2 | 529.6 ± 21 | 0 ± 8 | 0,4-0,5 | +++ |
| PP 5 | 97 | 16 3 | 83 ± 0.5 | 2158.2 ± 44 | 20 ± 9 | 2 | --- |

| | | | | | | | |
|---|---|---|---|---|---|---|---|
| Keys: § measured by DSC at second heating, *difference before and after 1 week of NGF incubation, +++ rhNGF-PP minimal interaction, --- rhNGF-PP maximum interaction. | | | | | | | |

From the data reported in Table 11, we concluded that the most suitable PP materials for the production of containers for NGF aqueous solutions were PP1, PP3 and PP4, namely those characterized by one or more of the following properties:
- ΔHm less than 95 J/g, preferably less than 90 J/g, more preferably less than 80 J/g and, preferably, ΔHm greater than 35 J/g, preferably greater than 40 J/g,
- water contact angle of F1 formulation greater than 90°(± 2), preferably greater than 95°(± 2),
- Delta AFM Roughness Sa (nm), before NGF incubation, lower than 10 nm, preferably lower than 5 nm,
- intensity of photoelectron emission of the nitrogen between 0,2 and 0,5 a.u. , preferably from 0,2 and 0,4 a.u.
properties measured according to the methods previously described.

### Example 6

### Measurement of the adsorption of rhNGF from aqueous formulations packaged in PP bottles vs siliconized glass vials (3 months stability studies)

We carried out a 3-months comparative stability test of rhNGF formulation F7, packaged in double siliconized glass vials 2R (2 ml, closed with an elastomer cap, manufactured by Soffieria Bertolini SpA) (V) or in PP bottles (10 ml, closed with Novelia^{®} System manufactured by Nemera) (B).

The composition of the formulation F7, prepared as formulations F1-F5, is reported in Table 12 below:

**Table 12: F7 formulation**

| Formulations | rh - NGF conc. (µg/ml) | Other ingredients | Notes |
|---|---|---|---|
| F7 | 20 | phosphate buffer (50 mM), methionine 0.020 mg/ml, NaCl 100 mM, water q.s. | pH 6.2 |

PP bottles (B) were made of PP3, one of the best PP according to the previous tests.

In this stability test, the closed glass vials (V) and PP bottles (B), containing the formulation F7, were kept at temperatures of 2-8°C, 25°C or 37°C and analyzed for NGF adsorption at time points of 1 week (1W), after 2 weeks (2W), 1 month (1 M), 2 months (2M) and 3 months (3M) versus a control formulation (Ctrl), namely F7 formulation but freshly prepared and analyzed immediately.

At each time point, the NGF content of at least two vials and two bottles was analyzed for some or all the temperatures. The Novelia^{®} PP bottles were manually pressed and drops were collected for analysis. The formulation samples were let to equilibrate at least 30 min at room temperature prior to measurement.

The NGF content in the solutions was measured by spectrofluorometric analysis on a Fluoromax spectrofluorometer (FluoroMax - 4) (autofluorescence emission).

To determine the protein concentration in the different NGF samples, calibration curves were prepared by UV-Vis absorbance and intrinsic fluorescence emission measures. rhNGF was diluted in the solution containing phosphate buffer (50 mM), methionine 0.020 mg/ml, NaCl 100 mM, water q.s. at pH 6.2, in the concentration range 5 - 40 µg/mL; three dilutions were performed for each concentration.

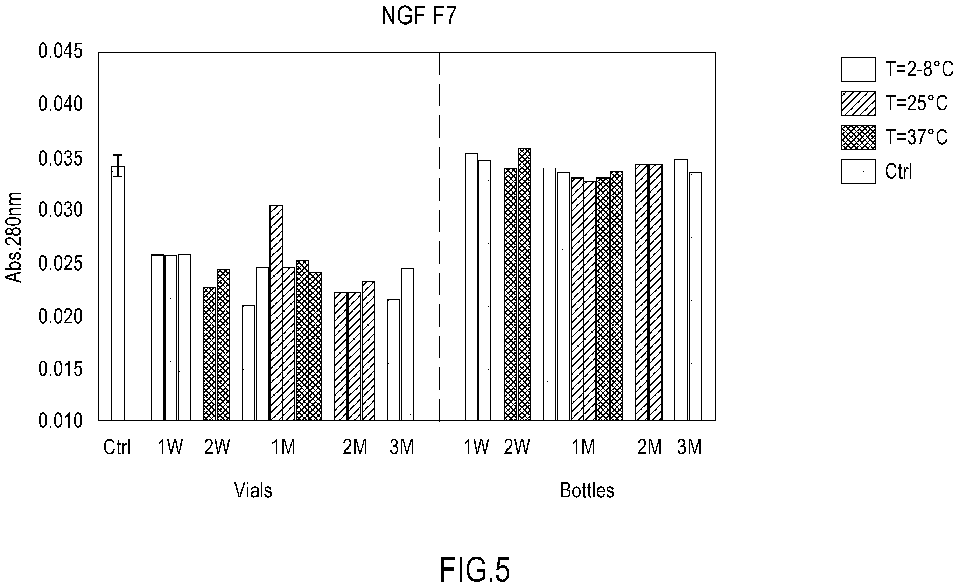

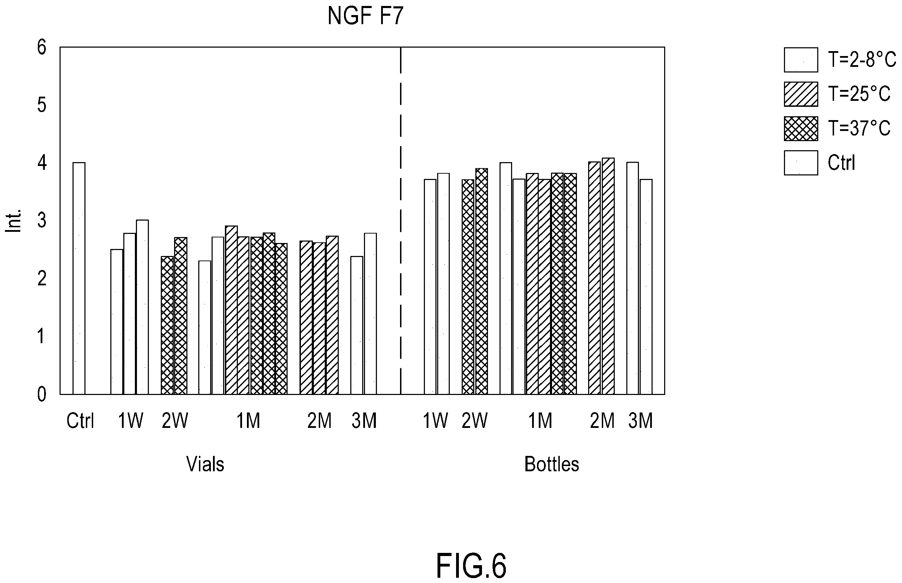

The NGF content of the samples (formulation F7 vs CTRL) assessed by spectroscopic analysis is shown in Figure 5 and by spectrofluorometric analysis in Figure 6.

The UV-Vis absorbance values at 280 nm were corrected for light-scatter (LS) background.

The bars with the same watermark refer to duplicates for the same time point. From these data, we observed that:
- for formulation F7, which only contained phosphate buffer and not a surfactant that prevents adsorption, it was evident that the NGF adsorbed more in the siliconized glass container than in the plastic container. This difference was particularly evident from the graphs of auto-fluorescence emission of tryptophan: for F7 the adsorption in the siliconized glass container was initially very rapid compared to the CTRL and then stabilized during the following 3 months (Figure 6);
- for both siliconized glass and PP containers, the NGF content was constant over a period of three months under all stability conditions. Both the auto-fluorescence of tryptophan and the Absorbance measurements indicated that there was no drop in NGF titer in the solution during the test, namely that NGF was stable under test conditions; in particular, by analyzing the wavelength of fluorescence emission of tryptophan, even in the container where the NGF adsorbs most (V, double siliconized glass vial) and with the most discriminating formulation (formulation F7 without surfactant), we did not notice any process of unfolding or misfolding of the NGF, which is indicative of the preservation of the biological activity of the protein. In conclusion, the analyses reported above were able to discriminate PP materials in terms of protein adsorption. Unexpectedly, it was showed with the above techniques that the PP plastic materials that adsorb less protein on their surface were hydrophobic materials, with a low degree of copolymerization with polyethylene and low crystallinity. The plastic material of choice PP3 adsorbed less protein at the solid-liquid interface than siliconized glass.

## Claims

1. A pharmaceutical package comprising a plastic container as primary packaging and a NGF aqueous formulation packaged therein, in which the plastic container has an inner surface in contact with the NGF aqueous formulation and an outer surface, wherein at least said inner contact surface is substantially made of at least a propylene - ethylene copolymer having a melting enthalpy ΔHm less than 95 J/g, measured by DSC according to the method reported in the description.

2. The package according to claim 1 wherein the propylene - ethylene copolymer has a melting enthalpy ΔHm less than 90 J/g, preferably less than 80 J/g measured by DSC according to the method reported in the description.

3. The package according to claim 2 wherein the propylene - ethylene copolymer has a melting enthalpy ΔHm greater than 35 J/g, preferably greater than 40 J/g, measured by DSC according to the method reported in the description.

4. The package according to any one of the previous claims wherein the propylene - ethylene copolymer has a melting temperature Tm lower than 160°C, preferably lower than 155°C, measured according to the DSC method reported in the description.

5. The package according to claim 4 wherein the propylene - ethylene copolymer has a melting temperature Tm higher than 130°C, more preferably higher than 140°C, measured according to the DSC method reported in the description.

6. The package according to any one of the previous claims wherein the propylene - ethylene copolymer is a random propylene - ethylene copolymer.

7. The package according to any one of the previous claims wherein the content of ethylene monomer in the propylene - ethylene copolymer is less than 40%, preferably less than 30%, and at least 5%.

8. The package according to any one of the previous claims wherein the container consists of the propylene - ethylene copolymer as defined in any one of the previous claims.

9. The package according to any one of the previous claims wherein the container is an ampoule, a vial, a bottle, a jar, a bag or a pouch, preferably is a vial or a bottle.

10. The package according to any one of the previous claims wherein the NGF aqueous formulation is an NGF aqueous solution, emulsion, suspension or lotion, preferably is a NGF aqueous solution, more preferably a NGF aqueous solution for ophthalmic use.

11. The package according to any one of the previous claims wherein, in the NGF aqueous formulation, NGF is present in amount from about 0.0001 % to about 0.5% w/v, preferably from about 0.001% to about 0.10% w/v, most preferably of about 0.002% w/v of the aqueous formulation.

12. The package according to any one of the previous claims wherein, in the NGF aqueous formulation, NGF is recombinant human NGF (rhNGF).

13. The package according to any one of the previous claims wherein the NGF aqueous formulation is buffered with a biologically acceptable buffer to a pH between 6.8 to 7.5, preferably to a pH of about 7.2.

14. The package according to any one of the previous claims wherein the NGF aqueous formulation in addition to rhNGF comprises trehalose, mannitol, Methocel, PEG 6000, L-methionine, phosphate buffer, water and has a pH of about 7.2.

15. The package according to any one of the previous claims that is a mono-dose or a multi-dose package.

16. A method for selecting a propylene-ethylene copolymer suitable for manufacturing at least the inner contact surface of the plastic container of the package according to any one of the previous claims that comprises:
- providing at least a propylene-ethylene copolymer sample
- measuring the melting enthalpy ΔHm of the propylene-ethylene copolymer sample by DSC according to the method reported in the description,
- selecting the propylene-ethylene copolymer as suitable if its melting enthalpy ΔHm is less than 95 J/g.

17. Use of a plastic container for packaging a NGF aqueous formulation, wherein the plastic container has an inner surface and an outer surface, wherein at least said inner surface is substantially made of at least a propylene - ethylene copolymer having a melting enthalpy ΔHm less than 95 J/g, measured by DSC according to the method reported in the description.
